# EUROPEAN PATENT APPLICATION

(11) **EP 3 871 622 A1**
(43) Date of publication of application: **01.09.2021**
(21) Application number: 21159513.7
(22) Date of filing: 26.02.2021
(51) Int. Cl.: A61B 17/34

(54) **ACCESS ASSEMBLY WITH RETENTION MECHANISM**

(30) Priority: 27.02.2020 US 202016803057
(71) Applicant: Covidien LP, Mansfield, MA 02048 (US)
(72) Inventor: TOKARZ, Christopher A., Wallingford, CT Connecticut 06492 (US); BUYDA, Oksana, East Haven, CT Connecticut 06513 (US); PATTISON, Douglas M., East Hartford, CT Connecticut 06108 (US); LOBO, Astley C., West Haven, CT Connecticut 06516 (US); ADINOLFI, Amanda M., Wallingford, CT Connecticut 06492 (US); DESJARDIN, Kevin, Prospect, CT Connecticut 06712 (US); MICKUS, Jason, Avon, CT Connecticut 06001 (US)
(74) Representative: Maschio, Antonio

(57) **Abstract**

A retention mechanism for a surgical access assembly includes a first member having a first semi-circular body with first and second ends and a second member having a second semi-circular body with first and second ends. The first end of the first member defines a first recess and the second end of the first member includes a first male connector. The first end of the second member includes a second male connector configured to be received within the first recess and the second end of the second member defines a second recess configured to receive the first male connector. When the first male connector is received within the second recess and the second male connector is received within the first recess, the first and second members are configured to frictionally engage a cannula of the surgical access assembly.

## Description

### FIELD

The present disclosure relates generally to a surgical access assembly having an anchor to securely engage tissue to prevent withdrawal of the surgical access assembly from the tissue, e.g., the abdominal wall, and, in particular, relates to an access assembly with a retention mechanism.

### BACKGROUND

Minimally invasive surgical procedures including both endoscopic and laparoscopic procedures permit surgery to be performed on organs, tissues and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula accessing the abdominal cavity to perform one or more surgical tasks. The cannula may incorporate a seal to establish a substantially fluid tight seal about the instrument to preserve the integrity of the pneumoperi toneum.

While minimally invasive surgical procedures have proven to be quite effective in surgery, limitations remain. For example, the cannula which is subjected to the pressurized environment, i.e., the pneumoperitoneum, may have a tendency to back out of the incision in the abdominal wall particularly during multiple manipulations of the instrument within the cannula. Conversely, during insertion and/or manipulation of instruments through the cannula, the cannula may become over-inserted, risking damage to the internal organs.

### SUMMARY

A retention mechanism for a surgical access assembly is provided. The retention mechanism includes a first member having a first semi-circular body with first and second ends, and a second member having a second semi-circular body with first and second ends. The first end of the first member defines a first recess and the second end of the first member includes a first male connector. The first end of the second member includes a second male connector configured to be received within the first recess and the second end of the second member defines a second recess configured to receive the first male connector. When the first male connector is received within the second recess and the second male connector is received within the first recess, the first and second members are configured to frictionally engage a cannula of the surgical access assembly.

The first and second recesses may each be frustoconical. The first and second recesses may correspond in shape to the respective first and second male connectors. The first and second members may be configured for releasable connection with one another. Each of the first and second members includes an inner surface, wherein each of the inner surfaces includes ridges. The first and second members may be formed of plastic or polymer.

In another aspect, the present disclosure relates to an access assembly with a retention mechanism. The access assembly includes a cannula having a proximal portion, a distal portion, and a length. An instrument valve is disposed on the proximal portion of the cannula, and an anchor mechanism is disposed on the distal portion of the cannula. The retention mechanism is movably disposed along the length of the cannula. The retention mechanism includes a first member including a first semi-circular body with first and second ends and a second member including a second semi-circular body with first and second ends. The retention mechanism is movable from a first position spaced a first distance from the anchor mechanism to a second position spaced a second distance from the anchor mechanism. The first distance is greater than the second distance.

The first end of the first member may be configured to engage the second end of the second member and the second end of the first member is configured to engage the first end of the second member. The first end of the first member may define a first recess and the second end of the first member may include a first male connector, and the first end of the second member may include a second male connector configured to be received within the first recess and the second end of the second member may define a second recess configured to receive the first male connector. The first male connector may be received within the second recess and the second male connector may be received within the first recess. The first and second members may be configured to frictionally engage a cannula of the surgical access assembly.

The first and second recesses of the retention mechanism may each be frustoconical. The first and second recesses of the retention mechanism may correspond in shape to the respective first and second male connectors of the retention mechanism. The first and second members may be configured for releasable connection with one another. Each of the first and second members of the retention mechanism may include an inner surface. Each of the inner surfaces may include ridges. The first and second members may be formed of plastic or polymer.

A method of securing an access assembly to tissue is also provided. The method includes receiving a proximal portion of a cannula of the access assembly through tissue, activating an anchor mechanism disposed on the proximal portion of the cannula, and advancing a retention mechanism disposed about the cannula towards the anchor mechanism.

The method may further include receiving the retention mechanism about the cannula. Receiving the retention mechanism about the cannula may include securing first and second members of the retention mechanism about the cannula about the cannula. Activating the anchor mechanism may include inflating a balloon anchor.

### BRIEF DESCRIPTION OF THE DRAWINGS

Various aspects and features of the present disclosure are described hereinbelow with references to the drawings, wherein:
FIG. 1 is a perspective view of an access assembly according to an aspect of the present disclosure including a retention mechanism;
FIG. 2 is a perspective view of the retention mechanism shown in FIG. 1;
FIG. 3 is a perspective view of a cannula of the access assembly and the retention mechanism shown in FIG. 1 prior to the retention mechanism being secured to the cannula;
FIG. 4 is a perspective view of the retention mechanism shown in FIG. 2, with parts separated;
FIG. 5 is a cross-sectional view of the access assembly and retention mechanism shown in FIG. 1 taken along lines 5-5 in FIG. 1;

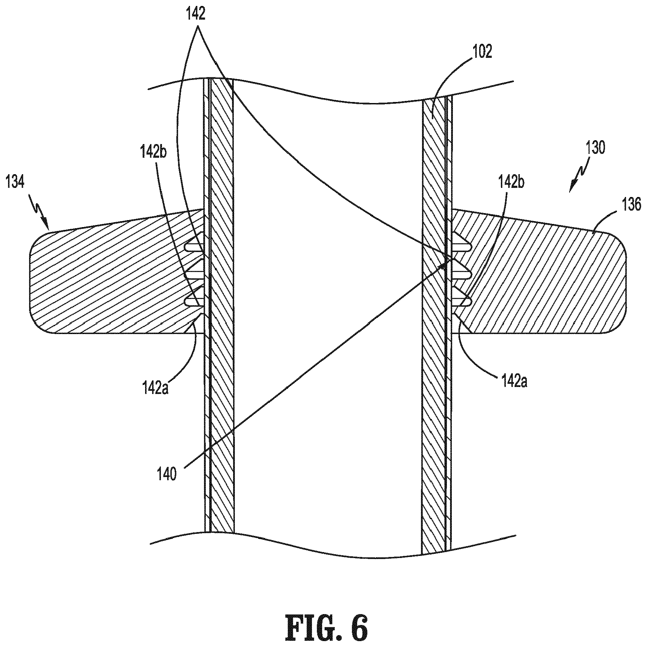

FIG. 6 is a cross-sectional view of the access assembly and retention mechanism shown in FIG. 5 taken along lines 6-6 in FIG. 5; and
FIG. 7 is a side view of the access assembly and retention mechanism shown in FIG. 1, secured to tissue.

### DETAILED DESCRIPTION

Particular access assemblies in accordance with the disclosure are described hereinbelow with reference to the accompanying drawings; however, it is to be understood that the disclosed access assemblies are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals refer to similar or identical elements throughout the description of the figures.

As used herein, the term "distal" refers to that portion of the instrument, or component thereof which is farther from the user while the term "proximal" refers to that portion of the instrument or component thereof which is closer to the user.

Access assemblies with obturators are employed during minimally invasive surgery, e.g., laparoscopic surgery, and provide for the sealed access of surgical instruments into an insufflated body cavity, such as the abdominal cavity. The access assemblies typically include an instrument valve housing mounted on a cannula tube, and include an obturator (not shown) inserted through the valve housing and cannula. The obturator can have a blunt distal end, or a bladed or non-bladed penetrating distal end and can be used to incise the abdominal wall so that the access assembly can be introduced into the abdomen. The handle of the obturator can engage or selectively lock into the instrument valve housing of the access assembly.

Access assemblies are employed to tunnel through an anatomical structure, e.g., the abdominal wall, either by making a new passage through the anatomical structure or by passing through an existing opening through the anatomical structure. Once the trocar assembly with the obturator has tunneled through the anatomical structure, the obturator is removed, leaving the access assembly in place. The instrument valve housing of the access assembly includes valves that prevent the escape of insufflation gases from the body cavity, while also allowing surgical instruments to be inserted into the cavity.

Many access assemblies include an anchor mechanism for preventing withdrawal of the access assembly. These anchor mechanisms may be in the form of an inflatable balloon. Alternatively, the access assemblies may be maintained in position with an expandable flange or other structure capable of being collapsed to facilitate insertion of the access assembly through the tissue and selectively expanded to prevent withdrawal of the access assembly from the tissue.

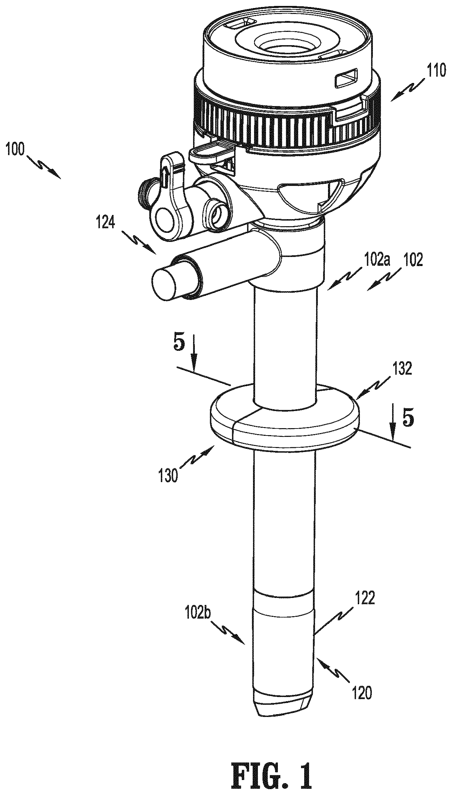

FIG. 1 illustrates an access assembly 100 suitable for use with a retention mechanism according to an exemplary device of the present disclosure. The access assembly 100 includes a cannula 102 and an instrument valve housing 110 supported to a proximal portion 102a of the cannula 102. Although shown including the instrument valve housing 110, it is envisioned that retention mechanisms in accordance with the disclosure may be incorporated into access assemblies without an instrument valve housing 110.

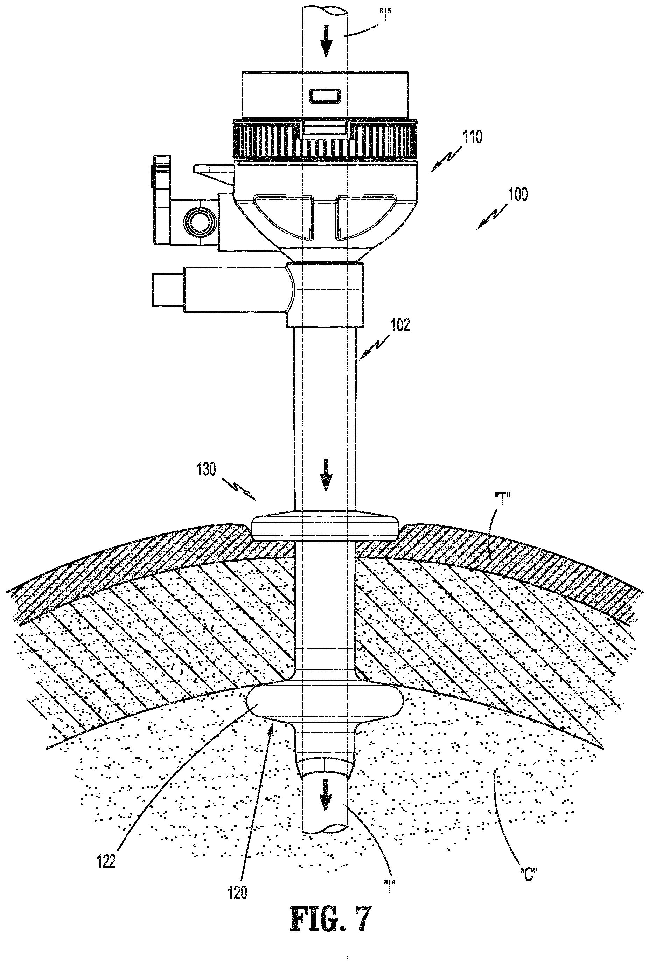

The access assembly 100 includes an anchor mechanism 120 supported on a distal portion 102b of the cannula 102. As shown, the anchor mechanism 120 includes a balloon anchor 122. The balloon anchor 122 includes an uninflated or collapsed condition (FIG. 1) and an inflated or expanded condition (FIG. 7). The balloon anchor 122 is inflatable through a port 124 supported on the proximal portion 102a of the cannula 102. The anchor mechanism 120 will only be described herein to the extent necessary to disclose the features of the retention mechanisms of the present disclosure. For detailed descriptions of the structure and function of exemplary anchor assemblies suitable for use with the access assemblies the present disclosure, please refer to U. S. Pat. Nos. 5,697,946, 7,691,089, and 10,327,809, and U.S. Pat. App. Pub. No. 2004/0138702. Although features of the retention mechanisms of the present disclosure will be shown and described with reference to a balloon anchor, it is envisioned that the retention mechanisms of the present of the disclosure may be used with access assemblies having various anchor mechanism, including, for example, a collapsible flange.

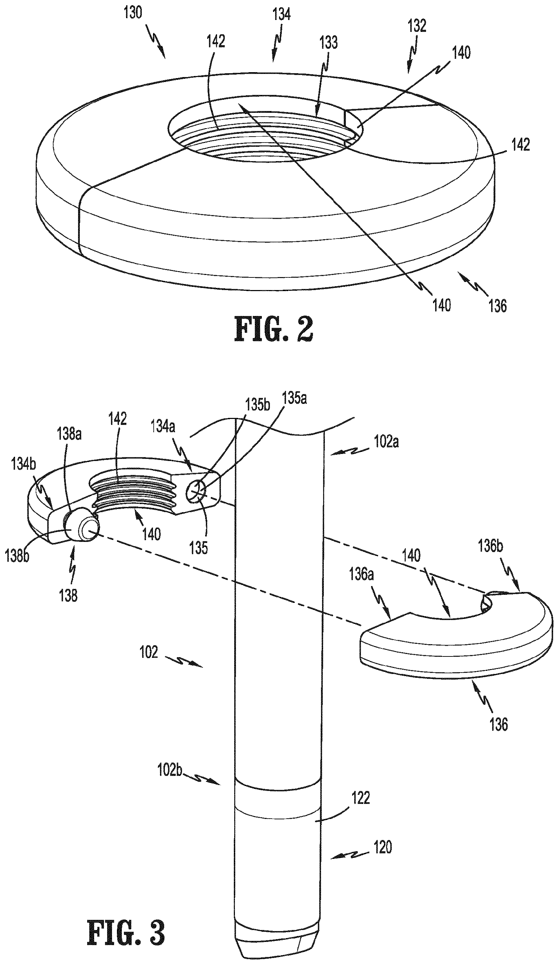

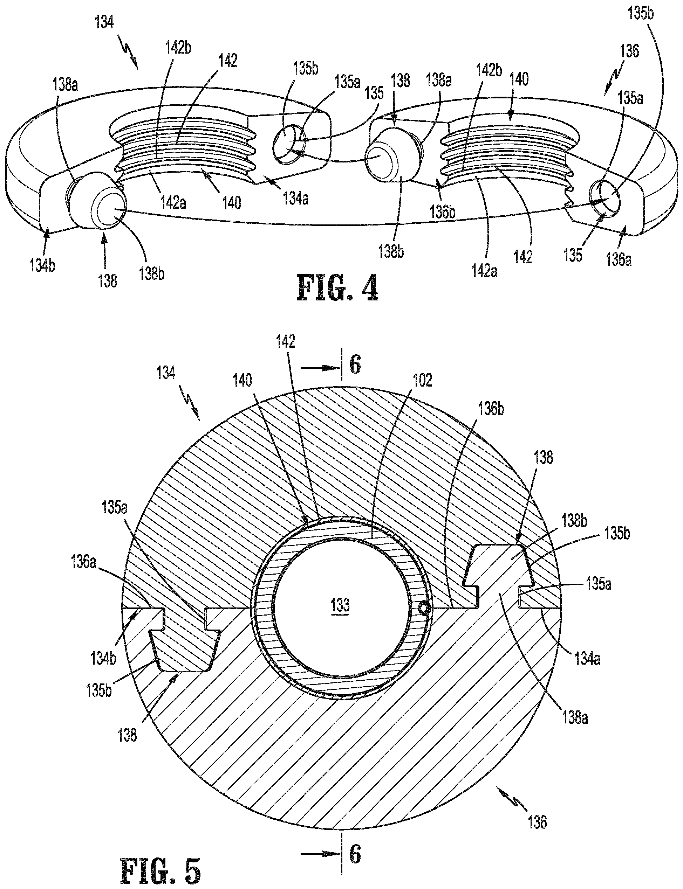

FIGS. 2-5 illustrate a retention mechanism 130 according to an exemplary example of the present disclosure. The retention mechanism 130 includes an annular body 132 configured to frictionally engage the cannula 102 of the access assembly 100 to prevent over-insertion of the access assembly 100 through the tissue during receipt and or manipulation of a surgical instrument (not shown) and/or specimen through the access assembly 100.

The annular body 132 of the retention mechanism 130 includes first and second ring halves 134, 136 that define an opening 133 configured to accommodate the cannula 102 of the access assembly 100 in a friction fit manner. The retention mechanism 130 may be formed of plastic, polymer, or other suitable material. The first and second ring halves 134, 136 may be substantially similar, as shown, to reduce the manufacturing cost and facilitate assembly of the retention mechanism 130. Although shown as being formed of two sections, it is envisioned that the retention mechanism may be formed from more than two sections.

Each of the first and second ring halves 134, 136 of the retention mechanism 130 each includes a first end 134a, 136a, and a second end 134b, 136b. The first ends 134a, 136a of the respective first and second ring halves 134, 136 define recesses 135. Each recess 135 includes a cylindrical portion 135a and a frustoconical portion 135b. Each of the second ends 134b, 136b include a male connector 138 configured to be received within the recess 135 in the first ends 134a, 136a of the respective first and second ring halves 134, 136. Each male connector 138 of the first and second ring halves 134, 136 includes a neck portion 138a and a head portion 138b disposed on a free end of the neck portion 138a. The head portion 138b of the male connector 138 is configured to be received within the frustoconical portion 135b of the recess 135 and the neck portion 138a of the male connector 138 is configured to be received within the cylindrical portion 135a of the recess 135. The head portion 138b of the male connector 138 is larger than the neck portion 138a of the male connector 138 and acts as a fastener to secure each male connector 138 within the respective opening 135.

Although shown with the recess 135 in each of the first ends 134a, 136a of the first and second ring halves 134, 136 and the male connector 138 on each of the second ends 134b, 136b of the respective first and second ring halves 134, 136, it is envisioned that a recess may be formed on both the first and second ends of the one of the respective first and second ring halves and a male connector may be formed both the first and second ends of the other of the respective first and second ring halves. In this manner, one of the first and second ring halves would define two recesses and the other of the first and second ring halves would include two male connectors.

An inner surface 140 of each of the first and second ring halves 134, 136 of the retention mechanism 130 may include ridges 142, as shown. The ridges 142 are configured to grip an outer surface of the cannula 102 to limit movement of the retention mechanism 130 relative to the cannula 102. The ridges 142 on each of the first and second ring halves 134, 136 may permit longitudinal movement of the retention mechanism 130 in a distal direction, e.g., towards the anchor mechanism 120. For example, and as shown, the ridges 142 include an angled distal facing surface 142a and a flat proximal facing surface 142b. The angled surface 142a accommodates flexing of the ridges 142 when a distal force is applied to the retention mechanism 130 to permit distal movement of the retention mechanism 130 relative to the cannula 102, and the flat surface 142b prevents flexing of the ridges 142 when a proximal force is applied to the retention mechanism 130 to prevent proximal movement of the retention mechanism 130 relative to the cannula 102. In this manner, and as will be described in further detail below, once the retention mechanism 130 is secured to the cannula 102, the retention mechanism 130 may be moved distally to sandwich the tissue, e.g., of the abdominal wall, between the anchor mechanism 120 (FIG. 7) and the retention mechanism 130 to ensure the access assembly 100 remains fixed relative to the tissue throughout a surgical procedure.

The retention mechanism 130 may be configured for releasable engagement of the cannula 102 of the access assembly 100. For example, the first and second ring halves 134, 136 may include finger grips or openings (not shown) to facilitate engagement by a user to permit pulling the first and second ring halves 134, 136 apart. Alternatively, the retention mechanism 130 may remain secured to the access assembly 100 and be disposed of along with the cannula 102 following the procedure. It is envisioned that the first and second ring halves 134, 136 may be fixedly secured to one another using adhesive, solvent, or in any other suitable manner.

FIG. 7 illustrates the access assembly 100 received through and secured to tissue "T", e.g., an abdominal wall. More particularly, after the distal portion 102b of the cannula 102 of the access assembly 100 is received through tissue "T", using an obturator (not shown) or other method, the balloon anchor 122 of the anchor mechanism 120 is inflated within a cavity "C" to prevent the cannula 102 from being withdrawn through the tissue "T". The retention mechanism 130 may be secured to the cannula 102 subsequent to distal portion 102b the cannula 102 being received through the tissue "T" or prior to the distal portion 102b of the cannula being received through tissue "T". The retention mechanism 130 may be provided to the surgeon already secured to the cannula 102 of the access assembly 100 or may be secured to the cannula 102 in the operating room prior to receipt of the cannula through the tissue "T".

By forming the retention mechanism 130 as two or more components, the retention mechanism 130 may be secured to the cannula 102 of the access assembly 100 after the access assembly 100 is manufactured, thereby reducing the likelihood of damage to the access assembly 100 and/or retention mechanism 130 during manufacturing.

The retention mechanism 130 is secured to the cannula 102 of the access assembly 100 by receiving the first and second ring halves 134, 136 about the cannula 102 with the male connector 138 of the first ring half 134 aligned with the recess 135 of the second ring half 136 and the male connector 138 of the second ring half 136 aligned with the recess 135 of the first ring half 134 and pressing the first and second ring halves 134, 136 together. Receipt of the head portion 138b of the male connectors 138 within the frustoconical portion 135b of the recesses 135 aligns the neck portion 138a of the male connectors 138 with the cylindrical portions 135a of the recesses 135 such that the first and second ring halves 134, 136 are locked relative to each other.

Following inflation of the balloon anchor 122 of the anchor mechanism 120, the retention mechanism 130 may be slid distally along the cannula 102 to squeeze or sandwich the tissue "T" between the anchor mechanism 120 and the retention mechanism 130. In this manner, the access assembly 100 is secured to the tissue "T" and the access assembly 100 is inhibited from longitudinal movement relative to the tissue "T" throughout insertion, withdrawal, and/or manipulation of a surgical instrument "I" through the access assembly 100.

Following a surgical procedure, the balloon anchor 122 of the anchor mechanism 120 may be deflated to permit withdrawal of the cannula 102 of the access assembly 100 from the tissue "T". As noted above, the retention mechanism 130 may be configured to be removed from the cannula 102 prior to or subsequent to removal of the access assembly from the tissue "T". Alternatively, the retention mechanism 130 may remain secured to and disposed with the cannula 102 of the access assembly 100.

Persons skilled in the art will understand that the devices and methods specifically described herein and illustrated in the accompanying drawings are non-limiting exemplary devices. It is envisioned that the elements and features illustrated or described in connection with the exemplary devices may be combined with the elements and features of another without departing from the scope of the disclosure. As well, one skilled in the art will appreciate further features and advantages of the disclosure based on the above-described devices. Accordingly, the disclosure is not to be limited by what has been particularly shown and described, except as indicated by the appended claims.

The invention may be described by reference to the following numbered paragraphs:
1. A retention mechanism for a surgical access assembly, the retention mechanism comprising: a first member having a first semi-circular body with first and second ends, the first end of the first member defining a first recess and the second end of the first member including a first male connector; and a second member having a second semi-circular body with first and second ends, the first end of the second member including a second male connector configured to be received within the first recess and the second end of the second member defining a second recess configured to receive the first male connector, wherein when the first male connector is received within the second recess and the second male connector is received within the first recess, the first and second members are configured to frictionally engage a cannula of the surgical access assembly.
2. The retention mechanism of paragraph 1, wherein the first and second recesses are each frustoconical.
3. The retention mechanism of paragraph 1, wherein the first and second recesses correspond in shape to the respective first and second male connectors.
4. The retention mechanism of paragraph 1, wherein the first and second members are configured for releasable connection with one another.
5. The retention mechanism of the paragraph 1, wherein each of the first and second members includes an inner surface, wherein each of the inner surfaces includes ridges.
6. The retention mechanism of paragraph 1, wherein the first and second members are formed of plastic or polymer.
7. An access assembly comprising: a cannula including a proximal portion and a distal portion and having a length; an instrument valve disposed on the proximal portion of the cannula; an anchor mechanism disposed on the distal portion of the cannula; and a retention mechanism movably disposed along the length of the cannula, the retention mechanism including a first member having a first semi-circular body with first and second ends and a second member having a second semi-circular body with first and second ends, the retention mechanism being movable from a first position spaced a first distance from the anchor mechanism to a second position spaced a second distance from the anchor mechanism, wherein the first distance is greater than the second distance.
8. The access assembly of paragraph 7, wherein the first end of the first member is configured to engage the second end of the second member and the second end of the first member is configured to engage the first end of the second member.
9. The access assembly of paragraph 8, wherein the first end of the first member defines a first recess and the second end of the first member includes a first male connector, and the first end of the second member includes a second male connector configured to be received within the first recess and the second end of the second member defines a second recess configured to receive the first male connector.
10. The access assembly of paragraph 9, wherein when the first male connector is received within the second recess and the second male connector is received within the first recess, the first and second members are configured to frictionally engage a cannula of the surgical access assembly.
11. The access assembly of paragraph 10, wherein the first and second recesses of the retention mechanism are each frustoconical.
12. The access assembly of paragraph 11, wherein the first and second recesses of the retention mechanism correspond in shape to the respective first and second male connectors of the retention mechanism.
13. The access assembly of paragraph 12, wherein the first and second members are configured for releasable connection with one another.
14. The access assembly of paragraph 8, wherein each of the first and second members of the retention mechanism includes an inner surface, wherein each of the inner surfaces includes ridges.
15. The access assembly of paragraph 8, wherein the first and second members are formed of plastic or polymer.
16. A method of securing an access assembly to tissue, the method comprising: receiving a proximal portion of a cannula of the access assembly through tissue; activating an anchor mechanism disposed on the proximal portion of the cannula; and advancing a retention mechanism disposed about the cannula towards the anchor mechanism.
17. The method of paragraph 16, further including receiving the retention mechanism about the cannula.
18. The method of paragraph 16, wherein receiving the retention mechanism about the cannula includes joining first and second members configured to be received about the cannula.
19. The method of paragraph 16, wherein activating the anchor mechanism includes inflating a balloon anchor.

## Claims

1. A retention mechanism for a surgical access assembly, the retention mechanism comprising:
a first member having a first semi-circular body with first and second ends, the first end of the first member defining a first recess and the second end of the first member including a first male connector; and
a second member having a second semi-circular body with first and second ends, the first end of the second member including a second male connector configured to be received within the first recess and the second end of the second member defining a second recess configured to receive the first male connector, wherein when the first male connector is received within the second recess and the second male connector is received within the first recess, the first and second members are configured to frictionally engage a cannula of the surgical access assembly.

2. The retention mechanism of claim 1, wherein the first and second recesses are each frustoconical.

3. The retention mechanism of claim 1 or claim 2, wherein the first and second recesses correspond in shape to the respective first and second male connectors.

4. The retention mechanism of any preceding claim, wherein the first and second members are configured for releasable connection with one another.

5. The retention mechanism of any preceding claim, wherein each of the first and second members includes an inner surface, wherein each of the inner surfaces includes ridges.

6. The retention mechanism of any preceding claim, wherein the first and second members are formed of plastic or polymer.

7. An access assembly comprising:
a cannula including a proximal portion and a distal portion and having a length;
an instrument valve disposed on the proximal portion of the cannula;
an anchor mechanism disposed on the distal portion of the cannula; and
a retention mechanism movably disposed along the length of the cannula, the retention mechanism including a first member having a first semi-circular body with first and second ends and a second member having a second semi-circular body with first and second ends, the retention mechanism being movable from a first position spaced a first distance from the anchor mechanism to a second position spaced a second distance from the anchor mechanism, wherein the first distance is greater than the second distance.

8. The access assembly of claim 7, wherein the first end of the first member is configured to engage the second end of the second member and the second end of the first member is configured to engage the first end of the second member.

9. The access assembly of claim 8, wherein the first end of the first member defines a first recess and the second end of the first member includes a first male connector, and the first end of the second member includes a second male connector configured to be received within the first recess and the second end of the second member defines a second recess configured to receive the first male connector.

10. The access assembly of claim 9, wherein when the first male connector is received within the second recess and the second male connector is received within the first recess, the first and second members are configured to frictionally engage a cannula of the surgical access assembly; preferably wherein the first and second recesses of the retention mechanism are each frustoconical.

11. The access assembly of claim 10, wherein the first and second recesses of the retention mechanism correspond in shape to the respective first and second male connectors of the retention mechanism; preferably wherein the first and second members are configured for releasable connection with one another.

12. The access assembly of claim 8, wherein each of the first and second members of the retention mechanism includes an inner surface, wherein each of the inner surfaces includes ridges; preferably wherein the first and second members are formed of plastic or polymer.

13. A method of securing an access assembly to tissue, the method comprising:
receiving a proximal portion of a cannula of the access assembly through tissue;
activating an anchor mechanism disposed on the proximal portion of the cannula; and
advancing a retention mechanism disposed about the cannula towards the anchor mechanism.

14. The method of claim 13, further including receiving the retention mechanism about the cannula; preferably wherein receiving the retention mechanism about the cannula includes joining first and second members configured to be received about the cannula.

15. The method of claim 13 or claim 14, wherein activating the anchor mechanism includes inflating a balloon anchor.
